# EUROPEAN PATENT APPLICATION

(11) **EP 1 629 788 A1**
(43) Date of publication of application: **01.03.2006**
(21) Application number: 03732175.9
(22) Date of filing: 19.05.2003
(51) Int. Cl.: A61B 17/425, G01N 1/02

(54) **A MICRO-HOLDING PIPETTE WITH AN OBLIQUE OPENING**

(71) Applicant: Xia, Jiahui, Changsha, Hunan Province 410078 (CN)
(72) Inventor: XIA, Jiahui, Nat. Lab of Medical Genetics of China, Changsha, Hunan 410078 (CN); LYU, Qifeng, Nat. Lab of Medical Genetics of China, Changsha, Hunan 410078 (CN)
(74) Representative: Roberts, Gwilym Vaughan
(86) International application number: PCT/CN2003/000363
(87) International publication number: WO 2004/100807

(57) **Abstract**

The present invention relates to a micro-holding pipette. The holding end of the micro-holding pipette is a oblique opening, i.e. the end surface of the opening of the holding end of the micropipette is at an acute angle with the ordinate axis of the holding end, which is less than 90°, preferably is from 25°to 85°. The present invention advantageously to pierces a pellucid zona from the side (not obverse surface) of a ovum or embryo by a micro injection needle or assistant needle for incubating, and without the deformation of the ovum or embryo due to the pressure. It is beneficial to various micro-processes. The novel micro-holding pipette and its operation can significantly prevent an embryo from being damaged and improve the healthy growth of the embryo.

## Description

### FIELD OF THE INVENTION

The present invention relates to a micro-holding pipette.

### BACKGROUND OF THE INVENTION

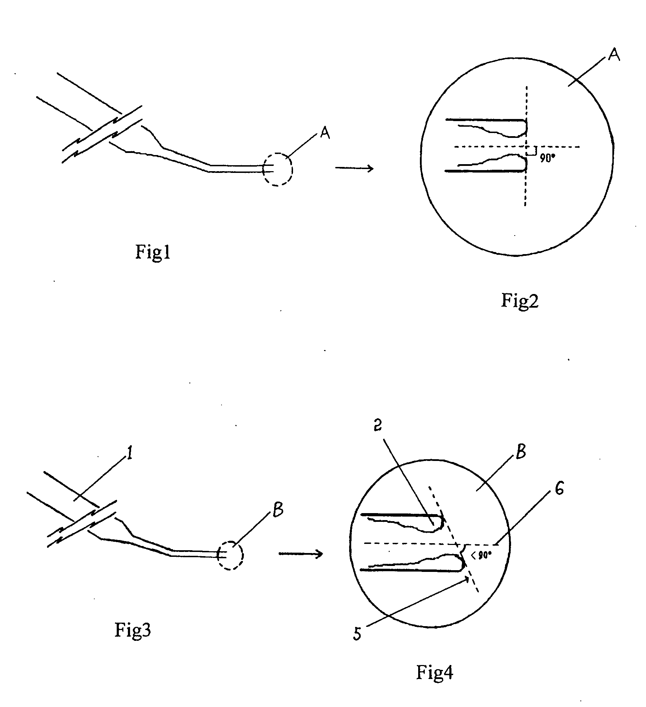

A micro-holding pipette is a micropipette that is used to hold or fix an ovum or embryo of human or animals in microscope by negative pressure. Until now, all kinds of micro-holding pipette have blunt ends, i.e. their surfaces are at a blunt angle (90°) with the ordinate axis of the holding end (Fig.1 and Fig.2). Pipettes are widely used in assisted reproductive technology (ART), for example, intracytoplasmic sperm injection (ICSI), assisted hatching (AH) and preimplantation genetics diagnosis (PGD). The ovum or embryo is held at a 9 o'clock position by a micro-holding pipette and another micropipette, a ICSI needle or a hatching needle opposite to the micro-holding pipette, pierces the zona pellucid (ZP) of the ovum or embryo at a 3 o'clock position during micro-processes. However, if the ICSI needle or hatching needle pierce ZP at other positions, for example 5 o'clock position, the ovum or embryo would be deformed or damaged due to the interlace mechanical pressure― the holding force and pierce force are not at the same axis. So an operator has to face more difficulties and need much more time in micro-processes with the traditional micro-holding pipette.

The cells of ovum or embryo will not be, but ZP loads the mechanical pressures when the opposite micropipette pierces the ZP at 4 o'clock to 6 o'clock positions firstly, then goes on to other steps. But with the traditional micro-holding pipette, the performance will be difficult because both the holding force and pierce force are not at the same axis as indicated above.

### DESCRIPTION OF THE INVENTION

Problems solved by the present invention are that: the ovum or embryo is held conveniently at a 45-past-8 o'clock to 7 o'clock position and the ZP is easily pierced or drilled at a 4 o'clock to 5 o'clock position by the opposite micropipette.
Characteristics of the present invention are that: the holding end of the MICRO-HOLDING PIPETTE is an oblique opening, i.e. the surface of the opening of the holding end of the micropipette is at an acute angle with the ordinate axis of the holding end, which is less than 90° and preferably is from 25° to 85°.

Advantages of the present invention are that: micropipettes or needles opposite to the MICRO-HOLDING PIPETTE can pierce or drill a ZP from the side not obverse surface of an ovum or embryo, the ovum or embryo do not deform due to the pressures, and it is beneficial to various micro-processes. The novel MICRO-HOLDING PIPETTE can significantly prevent damage to the embryo and improve the healthy growth of the embryo.

### BRIEF DESCRIPTION OF THE DRAWINGS

Fig.1 A traditional MICRO-HOLDING PIPETTE
Fig.2 A magnification of MICRO-HOLDING PIPETTE at 'A' site
Fig.3 The present invention
Fig.4 A magnification of the present invention at 'B' site
Fig.5 A hatching needle piercing at 5 o'clock
Fig.6 A hatching needle pierced at 5 o'clock

### EMBODIMENT

Fig.3 and Fig.4 show that: the holding end of the MICRO-HOLDING PIPETTE '1' is an oblique opening '2', i.e. the surface of the opening of the holding end '5' of the micropipette is at an acute angle with the ordinate axis '6' of the holding end, which is less than 90° , and preferably is from 25° to 85°.

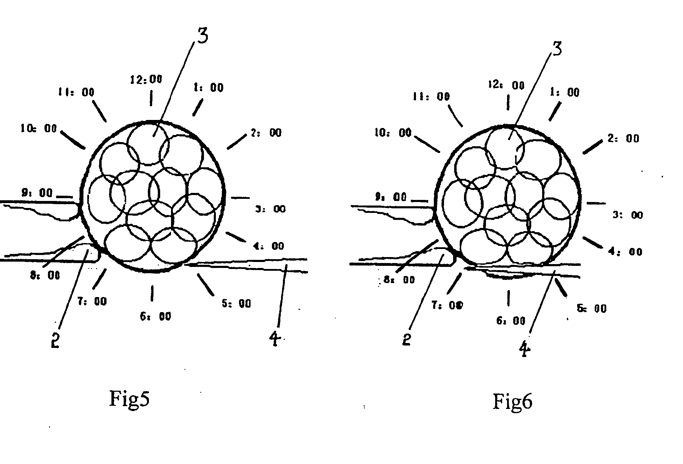

Fig.5 shows that: an embryo '3' is held at an 8 o'clock position and the hatching needle '4' easily pierces a ZP '3' of embryo at a 5 o'clock position (Fig.6).

During the manipulation, both the present invention and the hatching needle are at the same pressure axis that ensures the needle easily pierces or drill the ZP without deformation or damaging of the embryo. But the traditional MICRO-HOLDING PIPETTE does not have this advantage because the embryo has to be held at a 9 o'clock position.

There are several improvements in ZP mechanical drilling with the invention: □ it is more convenient and easy to pierce ZP and requires less time to drill ZP in micro-processes, □there are not squeezing and buffeting to the ovum or embryo which avoids mechanical harm, and Da novel method besides traditional methods can be used to ZP mechanical drilling, for example AH and PGD. Especially, the present invention is observed in ICSI.

## Claims

1. A micro-holding pipette with an oblique opening, **characterized in that** the surface of the opening of the holding end of the micropipette is at an acute angle with the ordinate axis of the holding end, which is less than 90°.

2. A micro-holding pipette according to claim 1, **characterized in that** the acute angle preferably is from 25° to 85°.
